# EUROPEAN PATENT SPECIFICATION

(11) **EP 4 651 841 B1**
(45) Date of publication and mention of the grant of the patent: **18.03.2026**
(21) Application number: 23853581.9
(22) Date of filing: 19.12.2023
(51) Int. Cl.: A61F 5/058

(54) **CRANIAL REMODELING ORTHOSIS**
KRANIALE REMODELLIERENDE ORTHESE
ORTHÈSE DE REMODELAGE CRÂNIEN

(30) Priority: 18.01.2023 CZ 20230018
(43) Date of publication of application: 26.11.2025
(73) Proprietor: INVENT MEDICAL GROUP, S.R.O., 70800 Ostrava (CZ)
(72) Inventor: ROSICKY, Jan, 73911 Frydlant nad Ostravici, Frydlant (CZ); ROSICKY, Jiri, 73911 Frydlant nad Ostravici, Frydlant (CZ); MARTINEK, Matej, 74601 Opava, Predmesti (CZ)
(74) Representative: Tomickova, Dana
(86) International application number: PCT/CZ2023/050089
(87) International publication number: WO 2024/153271

(56) References cited:
- EP-B1- 3 593 767
- WO-A1-2018/212632
- CA-A1- 2 996 246
- CN-U- 217 448 164
- US-A1- 2008 045 871

## Description

### Technical Field

The invention relates to a cranial remodeling orthosis, in particular for the treatment of head deformations in newborns, infants and toddlers.

### Background of the Invention

Cranial remodeling orthoses are used to secure or support remodeling of the wearer's head into the proper shape. These orthoses must be precisely adapted to the head of the particular wearer for both their comfort and proper function of the cranial orthosis.

For example, newborns, infants, and toddlers sometimes experience unwanted head deformation (usually from lying often in one position) that require prompt corrective treatment to correct the shape of the head. These deformations include the so-called plagiocephaly, which is a defect in the symmetry of the head, brachycephaly, which is a defect in the proportions of the head, or a combination of the two (referred to as asymmetrical brachycephaly), where the head exhibits both asymmetry and defective proportions. Plagiocephaly is manifested, for example, by asymmetrical flattening on the rear side of the head, prominence of the forehead on the side of the rear flattening, forward displacement of the ear on the side of the rear flattening, or asymmetry of the position of the face. Brachycephaly is manifested by a symmetrical flattening on the rear side of the head, a broader head, or a higher skull when viewed from above. The combination of these two types of deformation is then manifested by bilateral flattening of the rear side of the head, where one side of the head is flatter than the other, the forehead is displaced forward on the side of the greater flattening on the rear side of the skull, or the ear is displaced forward on the side of the greater occipital flattening.

These defects can be treated by wearing a cranial orthosis. Such an orthosis has an inner cavity to receive the head specifically designed to correspond to the shape of the head that the head should have at the end of treatment. Such an orthosis then directs the correction of the shape of the head during growth. This means that in some places the inner wall of the shell of the cranial orthosis is not in contact with the head, so head growth is supported in these places, while in places where the inner wall of the shell of the cranial orthosis is in contact with the head, head growth is limited. However, ensuring this ideal shape of the inner wall of the shell which ensures proper growth results in the fact that the cranial orthosis, by nature of its function, does not fit firmly on the wearer's head, wherein unwanted rotations of the orthosis relative to the head often occur, which results in less comfort for the wearer and less effectiveness of the cranial orthosis treatment.

Currently, there are several solutions to ensure a better fit of the cranial orthosis on the wearer's head. The problem of a better fit of the orthosis on the wearer's head is often solved by the fact that the shape of the inner wall of the shell is not adapted to the target shape that the head should have but only corresponds to a certain intermediate stage of growth towards the target shape. In such solutions, the shape of the inner cavity of the shell is adjusted such that the gap between the head and the inner wall of the shell is reduced in the places of deformations such that the orthosis does not allow such large unwanted rotational movements and at the same time growth is supported in the given place, i.e. the head is still not in contact with the inner wall of the shell in the place of deformation. The main disadvantage of this solution is the fact that the cranial orthosis has to be changed frequently, as after the head grows to the inner wall of the shell in the place of deformation, a new orthosis has to be created to again provide room for growth in the place of deformation. Thus, a large number of cranial orthoses must be used in the treatment process, corresponding to different intermediate stages in the growth to the target physiological shape of the head. The treatment thus requires a large amount of materials for the production of different shells, more specialist time is needed to plan the entire treatment, and the wearer often has to attend check-ups, making the treatment significantly more complicated and expensive.

These costs are reduced in solutions where the shell is made of a material that can be ground from the inside, wherein the specialist grinds the inside of the cranial orthosis in the course of the treatment such that the inner shape supports the growth in the places of deformation. Based on continuous check-ups, the inside of the orthosis is gradually ground to the ideal shape that the head should have. This process is technologically demanding and requires a high level of expertise, it is not possible to simply monitor and predict the course of the treatment, wherein it is always necessary to react to the actual shape of the wearer's head at the check-up. In addition, in the early stages of wear, the cranial orthosis is too heavy as it has to incorporate all the material that is gradually ground, which can lead to complications with the development of the neck and spine, possibly leading to their injury.

Another partial solution to the above-mentioned problems is a cranial orthosis with adjustable fastening, for example according to the document WO2019115965 A1. By means of the adjustable fastening of the helmet from the side, the helmet can be gradually opened as the head grows into the current volume of the inside of the shell. There is no need to modify the helmet in any way or to create new helmets all the time. However, such a solution can only be applied to some types of deformations and the effectiveness of such a solution is significantly limited. In addition, it is a structurally demanding solution and there is a risk of unwanted displacement of the clamping mechanism.

Another piece of the relevant state of the art is also the document CN 217448164 U, which discloses the principle of replacing the inner shell during the treatment. In this document, the inner shell is removably connected to an outer shell by means of protrusions fitting into through holes made in the wall of the outer shell on the left and on the right above an ear cutout. This document discloses a cranial remodeling orthosis as defined in the preamble of claim 1.

Other orthoses are described in documents CA 2996246 A1 and US 2008/045871 A1, where the inner shell is in a full contact and is stretching with a growing head during the whole period of wearing the orthosis. Further, In EP 3593767 B1, the inner shell and the outer shell are connected non-removably using an adhesive.

For the above reasons, it is desirable to develop a cranial remodeling orthosis that sits firmly and comfortably on the wearer's head, providing correct treatment for a wide range of different head deformations during the whole period of wearing the cranial remodeling orthosis.

### Summary of the Invention

The above shortcomings are eliminated by the cranial remodeling orthosis of the invention comprising an outer shell and an inner shell, wherein the inner wall of the outer shell defines an inner cavity of the outer shell for insertion of the wearer's head, wherein the inner shell is removably connected to the outer shell and defines a reduced inner cavity for insertion of the wearer's head that has a smaller volume than the inner cavity of the outer shell. The reduced inner cavity corresponds to a certain intermediate stage of growth between an initial outer shape of the wearer's head and a target outer shape of the wearer's head. The inner shell is removably connected to the outer shell in at least 2 different places distributed around a margin of the inner shell.

The advantage of the present invention of the cranial remodeling orthosis is that it sits firmly and comfortably on the wearer's head while providing proper treatment for a wide range of different head deformations, throughout the whole period of wearing such a cranial remodeling orthosis.

Thus, the shell of the cranial orthosis of the invention is composed of at least two pieces, the outer shell, and the inner shell. The outer shell refers to the basic load-bearing part of the cranial orthosis, which defines the outer shape of the orthosis and ensures sufficient firmness and structural stability of the orthosis. The outer shell comprises the inner wall of the outer shell, which defines the inner cavity of the outer shell and forms the basis for defining the inner cavity of the cranial orthosis, which is adapted to contain the wearer's head. The inner cavity of the outer shell does not mean a cavity within the wall of the outer shell but a cavity that is defined by the shape of the outer shell and that to a certain extent defines the space for containing the wearer's head, wherein the resulting space of the cranial orthosis for containing the wearer's head is then represented by the inner cavity of the cranial orthosis. In various embodiments, the inner wall of the outer shell may be provided with a lining or additional layers ensuring, for example, greater comfort or safety. The outer shell may be implemented as a thin-walled shell comprising only one envelope or layer or may comprise an inner and outer envelope with a mesh 3D structure between them to reinforce the structure, or a combination of these variants. In some embodiments, the inner wall of the outer shell may represent the inner wall of the cranial orthosis, in other words, it may directly define the inner cavity of the cranial orthosis that is adapted to contain the wearer's head. In other embodiments, the cranial remodeling orthosis may contain additional components placed within the outer shell that form the entire inner wall of the cranial orthosis and thus the inner cavity of the cranial orthosis that is adapted to contain the wearer's head, or it forms it only partially together with the inner wall of the outer shell. Thus, the inner cavity of the outer shell may not correspond to the inner cavity of the cranial orthosis in some embodiments, or the inner wall of the outer shell may not correspond to the inner wall of the cranial orthosis in some embodiments.

The inner cavity of the cranial orthosis for insertion of the wearer's head means the space within the cranial orthosis that the cranial orthosis surrounds in shape and that is adapted to contain the wearer's head, this space thus roughly corresponds to the outer shape of the wearer's head. The inner cavity of the cranial orthosis is defined by the shell or other layers placed on the inner wall of the shell, wherein its orifice corresponds to the marginal contours of the shell that in cranial remodeling orthoses run above the eyebrows, then on both sides of the head around the ears and meet in the region of the occipital bone. With its inner shape, the cranial remodeling orthosis, or rather the inner cavity of the orthosis, roughly corresponds to the shape of the skull in the regions of the frontal bone, parietal bones, and occipital bone. From the inner side, the inner shell and/or the outer shell may comprise a lining or additional layers placed on the inner walls of the shells for greater comfort or breathability of the cranial orthosis. These additional layers naturally affect the shape of the inner wall of the cranial orthosis and therefore the shape of the inner cavity of the cranial orthosis for containing the head, wherein these additional layers must be considered in the design of the inner and outer shell.

The inner shell can be connected to the outer shell removably in various ways. For example, the inner shell may be connected directly to the inner wall of the outer shell such that the inner shell and the outer shell form one continuous piece. In such an embodiment, the inner shell is connected to the outer shell in at least 2 places around its margin by bridges, preferably in at least 4 places around its margin, more preferably around its entire margin. These pins/bridges are thin and fragile compared to the rest of the structure of the shells, wherein when the inner shell is twisted relative to the outer shell, they break and the inner shell can thus be removed from the inner cavity of the outer shell. In other embodiments, the inner shell may extend over the margins of the outer shell, wherein in these embodiments the inner shell may also be connected to the outer side of the outer shell. In addition, the inner shell may be connected to the outer shell by gluing, double-sided adhesive tape, or by a mechanical connection such as a groove-projection type, or via an elastic stop. In some embodiments, the inner shell may be connected to the outer shell via lining. In other embodiments, the inner shell may be connected to the inner wall of the outer shell. Thus, a removable connection between the inner and outer shells generally means such an implementation of their connection that allows separation of the inner shell from the outer shell without significant damage to the outer shell that would limit the functionality of the cranial orthosis.

Furthermore, the outer shell may be composed of multiple parts that are connected by a clamping mechanism when placed on the wearer's head. In these embodiments, the description of the inner wall of the cranial orthosis, the inner wall of the outer shell, the inner cavity of the outer shell, and the inner cavity of the cranial orthosis refers to the shell in the assembled position where all pieces of the outer shell are put together.

In the context of the invention, the cranial orthosis has a variable inner cavity of the cranial orthosis, wherein the size of the inner cavity of the cranial orthosis may change upon removal of the inner shell from the outer shell, or by changing one inner shell for another. In this context, two inner cavities of the cranial orthosis are defined: target and reduced. The reduced cavity of the cranial orthosis is defined by the reduced inner wall of the cranial orthosis and the target cavity of the cranial orthosis is defined by the target inner wall of the cranial orthosis, wherein the reduced cavity of the cranial orthosis has a smaller volume than the target cavity of the cranial orthosis, or the target cavity is adapted to contain a wearer's head with a larger volume. Thus, in general, the target and reduced cavities of the cranial orthosis correspond to different stages of head growth during the remodeling, where the reduced cavity corresponds to the growth intermediate stage in the growth of the head to the target shape of the wearer's head, which corresponds to the target inner cavity of the cranial orthosis. The reduced wall of the cranial orthosis may be formed together by the inner and outer shells or entirely by the inner shell, the target wall of the cranial orthosis may be formed together by the inner and outer shells, entirely by the inner shell, or entirely by the outer shell. Additional layers, such as, for example, the lining on the inner or outer shell, affect the shape of the reduced or target inner wall, or cavity, wherein the implementation of the inner shell and/or outer shell must be adapted in their shape to the given additional layers such that together they form the required shape of the inner wall of the cranial orthosis and, therefore, of the inner cavity of the cranial orthosis.

The reduced inner cavity of the cranial orthosis corresponds to the volume of the target inner cavity of the cranial orthosis reduced by the volume taken from the target inner cavity volume by the inner shell. Thus, the reduced inner cavity means the target inner cavity of the cranial orthosis reduced by a certain volume defined by the inner shell inserted into the inner cavity of the outer shell.

Preferably, the inner shell is removably connected to the inner wall of the outer shell. In these embodiments of the invention, the inner wall of the outer shell has a generally concave or convex shape adapted to the shape of the head and defining the inner cavity of the outer shell that also corresponds to the target inner cavity of the cranial orthosis. The inner shell preferably has a shape roughly following the concavity of the inner wall of the outer shell in the given place. The shape of the inner shell is more flattened relative to the shape of the region of the inner wall of the outer shell that the inner shell covers with growing difference between the target inner cavity and the reduced inner cavity, or growing volume of the inner cavity of the outer shell enclosed by the inner shell. The inner shell preferably continuously follows the wall of the inner cavity of the outer shell, wherein the transition between the inner wall of the outer shell and the surface of the inner shell is gradual and smooth for maximum comfort of the wearer. In these embodiments, the reduced inner wall of the cranial orthosis is formed together by the surface of the inner shell, or inner wall of the inner shell, and the inner wall of the outer shell, wherein after the inner shell is removed, the target inner wall of the cranial orthosis is formed by the inner wall of the outer shell.

In other alternative preferred embodiments, the cranial remodeling orthosis of the invention is a part of an assembly that further comprises at least one other inner shell for the removable connection to the outer shell, wherein each of the at least two inner shells defines a different reduced inner cavity having a different volume. In these embodiments, the assembly comprises two mutually replaceable inner shells: the first inner shell and the second inner shell, both of which are adapted for removable connection with the outer shell. The first inner shell may at least partially form the reduced inner wall of the cranial orthosis, wherein after replacement, the second inner shell at least partially forms the target inner wall of the cranial orthosis. In such an embodiment of the invention, the inner cavity of the outer shell does not represent the inner cavity of the cranial orthosis, as it is not adapted to contain the wearer's head. In other embodiments, the first inner shell may form the first reduced inner wall of the cranial orthosis, the second inner shell may form the bulkier second reduced inner wall of the cranial orthosis, and the target inner wall of the cranial orthosis may be formed by the inner wall of the cranial orthosis. The assembly of the cranial remodeling orthosis of this embodiment of the invention comprises one outer shell and at least two inner shells, wherein these inner shells are adapted to define, when connected to the outer shell, different inner cavities of the cranial orthosis corresponding to different phases of growth of the wearer's head.

Preferably the inner shell comprises a perforated surface of the inner shell. The surface of the inner shell may be rigid or flexible, wherein increased flexibility may be achieved by perforations or material selection. Preferably the inner shell further comprises a mesh 3D structure filling at least partially the volume between the surface of the insert and the inner wall of the cranial orthosis. The inner shell comprises a surface of the inner shell, wherein the surface of the inner shell may be continuous or perforated. The inner shell may be implemented only as a specifically shaped wall defining the surface of the insert, which is adapted for removable connection to the outer shell at its margin. In other embodiments, the inner shell may comprise a 3D mesh structure that at least partially fills the space between the surface of the inner shell and the inner wall of the cranial orthosis that is covered by the inner shell, wherein the 3D mesh structure may also form the surface of the inner shell.

The mesh 3D structure comprises shaped openings distributed in its volume, the specific implementation of which can be used to modify the flexibility or stiffness of the inner shell, or only a part of it. Depending on the specific shape, distance, and size, the openings modify the stiffness of the given region and give it a different directional expansion. The openings are meant to be volume cutouts of a specific shape in the volume of the mesh 3D structure. The stiffness of the mesh 3D structure is determined by the specific shape, distance, and size of the openings located in the given volume. The openings are distributed in the volume of the mesh 3D structure such that the stiff structure filling the volume between them has the structure of a three-dimensional (3D) mesh. In the case where the openings are smaller, have a shape that provides a greater stiffness to the surrounding structure, and/or are at a distance from each other, thicker structures of the material that fills the space between the openings are achieved, thereby ensuring a higher stiffness of the mesh 3D structure. By thicker 3D printed structures are meant structures with a larger cross-section in the place between the openings and with a higher volume representation in proportion to the volume representation of the openings. If the openings have a larger shape that provides a lower stiffness to the surrounding structure, and/or are located close together, weaker structures of the material that fills the space between the openings are achieved, thereby ensuring a lower stiffness of the mesh 3D structure. By weaker 3D printed structures are meant structures with a smaller cross-section in the place between the openings and with a smaller volume representation relative to the volume representation of the openings.

The cranial remodeling orthoses of the invention, in various embodiments of the inner and outer shells, form the target inner wall defining the target cavity of the cranial orthosis and the reduced inner wall defining the reduced cavity of the cranial orthosis. The target and reduced cavity of the cranial orthosis can also be understood in the context of the invention as cavities defined not only by the cranial remodeling orthosis but also by the outer shape of the wearer's head, specifically the initial shape of the wearer's head. In other words, in general, the inner cavity of the cranial orthosis can be understood as the cavity between the outer shape of the wearer's head and the inner wall of the cranial orthosis. Thus, the target inner cavity of the cranial orthosis may be understood as the cavity between the initial outer shape of the wearer's head and the target inner wall of the cranial orthosis, and the reduced inner cavity of the cranial orthosis may be understood as the cavity between the initial outer shape of the wearer's head and the reduced inner wall of the cranial orthosis. In this sense, the reduced inner cavity of the cranial orthosis may be 10 to 90% smaller, preferably 40 to 60% smaller, than the target inner cavity of the cranial orthosis.

The inner shell of the invention is preferably adapted to be placed in the inner cavity of the outer shell of the cranial orthosis in a region corresponding to a region of significant deformation on the wearer's head, i.e. a region where the current shape of the wearer's head is significantly smaller than the normal, physiological shape of the head that is the target of the treatment with the cranial remodeling orthosis, wherein growth is to be supported by the orthosis in this place, while in other regions of the head growth may be limited by the orthosis. Thus, the inner wall of the inner shell corresponds to the shape of the head at a certain intermediate stage between the current shape of the head and the target physiological shape of the head.

For the invention to function properly, it is necessary that the reduced inner wall corresponds at least partially to the target inner wall of the cranial orthosis when they are centered. The difference in their shape is required only in the place of deformation where the reduced inner wall at least partially reduces the space between the wearer's head and the target inner wall of the cranial orthosis. The inner shell must form the reduced inner wall such that it corresponds to the intermediate stage of growth of the head in the place of deformation, while in the other regions of the head it is undesirable to support growth, since they have a shape that already corresponds to the target outer shape of the head, wherein in these other regions (which are not part of the deformation) the inner wall of the cranial orthosis is unchanged in both the reduced and the target version.

Preferably, the inner shell is connected to the outer shell in at least 2 different places distributed around the margin of the inner shell. Preferably, the inner shell is connected to the inner wall of the cranial orthosis along most of its margin. In embodiments where the inner shell is connected to the inner wall of the outer shell, preferably the inner shell has a rounded margin and it is preferable that the connection to the outer shell is located around the margin of the inner shell to ensure the most stable adhesion possible of the inner shell to the inner side of the outer shell, thereby preventing the formation of gaps between the shells at the margins. Similarly, in embodiments where the inner shell is connected to the outer shell in other regions of the outer shell, such as around their margin at the orifice of the inner cavity of the outer shell or from the outer side of the outer shell, it is preferable even here for the connections to be located around the margin of the inner shell. Thus, the connections are not in the places where the inner shell and/or outer shell form the inner wall of the cranial orthosis, wherein they do not structurally affect the structure of the shells in those places, and the structure of the inner wall of the cranial orthosis can be entirely focused on the function and comfort of the cranial remodeling orthosis.

Preferably the inner shell is made of 3D printed material. The inner shell and outer shell are preferably made by 3D printing technology such as SLA, SLS, FDM, MJF, DLP, 3DP, PJF, CLIP technology. The material for production of the inner shell and/or outer shell can be, for example, polymers PA, ABS, PLA, PE, PP, CPP, HPP, PC, PETG, photopolymers, elastomers (TPU, TPA, TPE, silicones), and other materials suitable not only for the above-mentioned 3D printing methods. Preferably, a thermoplastic elastomer such as thermoplastic polyamide (TPA), thermoplastic polyurethane (TPU), or a copolymer blend (TPE) is used to produce the 3D mesh structure.

The method of designing and producing the cranial remodeling orthosis of the invention comprises the steps of:
a) obtaining a model of the outer shape of the wearer's head,
b) designing a model of the cranial remodeling orthosis based on the model of the outer shape of the wearer's head comprising a step of determining the target outer shape of the wearer's head based on the model of the outer shape of the wearer's head and a subsequent step of determining the target inner wall of the cranial orthosis defining the target inner cavity of the cranial orthosis,
c) producing the cranial orthosis,
wherein the step of designing the model of the cranial orthosis (b) further comprises a step of determining the reduced inner wall of the cranial orthosis defining the reduced inner cavity of the cranial orthosis comprising a step of comparing the model of the outer shape of the wearer's head with a representation of the target outer shape of the wearer's head, wherein the step of designing the model of the cranial orthosis (b) further comprises a step of designing the inner shell based on the step of determining the reduced inner wall of the cranial orthosis, wherein the reduced inner wall of the cranial orthosis is at least partially formed by the inner shell, and the step of producing the cranial orthosis (c) comprises a step of producing the inner shell.

The initial step of the method of designing and producing the cranial remodeling orthosis of the invention is the step of obtaining the model of the outer shape of the wearer's head, or a 3D model based on the actual anatomical shape of the wearer's head, wherein this model can be obtained in several ways ranging from various scanning methods to entering the measured data into a computer device that designs the 3D model of the head based on the data. The wearer means the person for whom the cranial remodeling orthosis is intended. The most common method of obtaining the 3D model based on the anatomical shape of the wearer's head is a direct 3D scan of the wearer's head. During the 3D scan, the data obtained from the 3D scan of the wearer's head is transformed into a polygonal grid of defining points with a density of at least 10 points per cm². Another method is to make a physical model of the wearer's head, for example from a cast of the wearer's head, and a subsequent 3D scan of the created physical model. In some cases, it is advisable to modify the physical model of the head before the 3D scan. Other methods of obtaining the model of the outer shape of the wearer's head are, for example, digitizing the inner area of an existing cranial orthosis or making a physical model of the wearer's head from an existing cranial orthosis and subsequently digitizing the outer area of the obtained physical model of the head, which can be modified before the digitization. Furthermore, the model of the outer shape of the wearer's head can be obtained by first measuring the wearer's head, for example, its circumference, width, length, diagonal dimensions of the head, and these data are entered into a computer device based on user input, which designs a 3D model based on these data. The obtained 3D model based on the anatomical shape of the wearer's head is subsequently uploaded from the scanning device to the computer device, where it is further processed within the method of designing according to the invention.

The step of designing the model of the cranial orthosis follows, which comprises the steps of determining the target outer shape of the wearer's head, determining the target inner wall of the cranial orthosis, the step of determining the reduced inner wall of the cranial orthosis, and the step of designing the inner shell. The step of determining the target outer shape of the wearer's head is performed based on the input parameters that comprise the model of the outer shape of the wearer's head. The model of the outer shape of the wearer's head can be modified before further steps are taken to better adapt to the specific needs of the wearer. Thus, the model of the outer shape of the wearer's head may mean a physical representation, a modified physical representation, a digital representation, or a modified digital representation of the outer shape of the wearer's head. Other input parameters may represent age, gender, type, and severity of the deformity, the wearer's medical data, planned length of the cranial orthosis treatment, width of the head in various places, length of the head, circumference of the head, shape/circumference percentile of the growth of the head, diagonal dimensions of the head, or other parameters representing the initial state of the wearer's head. Thus, the input parameters define the initial state of the wearer, in particular, the data that define the current deformation and affect the process of the remodeling of the head are important.

The target outer shape of the head is designed based on these input parameters, wherein it is based on the shape of the head that the head should have, i.e. the physiological shape of the head, taking into account the planned period of wearing the cranial remodeling orthosis by the wearer. The initial shape of the wearer's head, or the model of the outer shape of the wearer's head, shows the regions and types of deformations that need to be corrected. The most common corrections are a defect in the symmetry of the head - plagiocephaly, a defect in the proportions of the head - brachycephaly, or a combination of a defect in symmetry and a defect in proportions - a so-called combined deformity. In the centered position of the 3D model of the initial outer shape of the wearer's head and the 3D model of the target outer shape of the wearer's head, in some regions the 3D model of the target outer shape of the wearer's head is located above the area of the 3D model of the initial shape of the wearer's head, wherein in these regions the growth of the head will be supported, in other regions the area of the 3D model of the target outer shape of the wearer's head merges with the area of the 3D model of the initial outer shape of the wearer's head, wherein in these regions the growth will be at least partially limited.

The step of determining the target inner wall of the cranial orthosis is based on the step of determining the target outer shape of the wearer's head, wherein the resulting shape of the target inner wall is determined to achieve the target outer shape of the wearer's head. In some embodiments, the shape of the target inner wall of the cranial orthosis may directly correspond to the target outer shape of the wearer's head. In other embodiments, their shapes may differ, for example, the target inner wall of the cranial orthosis may be smaller in some regions, or may represent a less bulky shape of the head, wherein in these "reduced" regions the target inner wall of the cranial orthosis comprises flexible regions that may provide a firmer and more stable fit of the orthosis on the head, while at the same time, thanks to the flexibility, these regions support the growth of the head to a certain extent.

The step of designing the model of the cranial orthosis further comprises a step of designing the outer shell. It is designed to form a sufficient structural basis for the specific implementation of the inner walls of the cranial orthosis. In embodiments where the inner wall of the outer shell forms the target inner wall of the cranial orthosis, it is evaluated whether any additional layers, such as a lining, will be implemented on the outer shell. These layers and additional elements in the inner cavity of the orthosis affect the shape of the inner cavity of the cranial orthosis for insertion of the wearer's head, whether target or reduced, and therefore affect its function as a remodeling tool. All this information must be processed to create a customized lining and shell that together form the ideal inner wall of the cranial orthosis that will be both comfortable and will represent the ideal remodeling tool to achieve a physiological shape of the head. Thus, in some embodiments, the outer shell or surface thereof may correspond to the shape of the target inner wall of the cranial orthosis increased by a thickness corresponding to the given additional layer, such as a lining, such that together they form a shape corresponding to the target inner wall of the cranial orthosis. In other words, the inner wall of the outer shell does not have to correspond to the shape of the target inner wall of the cranial orthosis, wherein the resulting shape of the target inner wall of the cranial orthosis is achieved by placing the additional layer on the inner wall of the outer shell. In embodiments without additional layers, the shape of the inner wall of the outer shell may directly correspond to the target outer shape of the wearer's head.

Next, the step of designing the model of the cranial orthosis comprises the step of determining the reduced inner wall of the cranial orthosis. The step of determining the reduced inner wall of the cranial orthosis occurs similarly to the step of determining the target inner wall of the cranial orthosis, except that it is not based on the target outer shape of the wearer's head but on the shape of a certain intermediate stage of growth between the initial outer shape of the wearer's head and the target outer shape of the wearer's head. Thus, it can be said that the reduced inner wall of the cranial orthosis is designed based on a reduced model of the outer shape of the wearer's head that corresponds to this intermediate stage of growth. Therefore, the step of determining the reduced inner wall of the cranial orthosis comprises the step of comparing the model of the outer shape of the wearer's head with a representation of the target outer shape of the wearer's head. The representation of the target outer shape of the wearer's head may mean, in various embodiments, the target outer shape of the wearer's head, the target inner wall of the cranial orthosis, and the target inner cavity of the cranial orthosis, in digital or physical form. In some embodiments, the cranial remodeling orthosis with the target inner wall of the cranial orthosis may be formed in advance of the design and production of the inner shell, wherein in such cases, the representation of the target outer shape of the wearer's head may already be this formed target inner wall of the cranial orthosis. In such embodiments, for example, the appropriate reduced inner wall of the cranial orthosis, or inner shell, may be determined by a trial-and-error method using preproduced inner shells, where the best fit is tested in the step of comparing. The step of determining the reduced inner wall of the cranial orthosis and the step of designing and producing the inner shell can thus occur simultaneously, or they can completely merge into one step. Preferably, however, this step of comparing occurs in a digitized form where the models are centered and the step of determining the reduced inner wall of the cranial orthosis occurs based on the space between the area of the digital 3D model of the initial shape of the wearer's head and the digital 3D model representation of the target outer shape of the wearer's head.

In some embodiments, the shape of the reduced inner wall of the cranial orthosis may directly correspond to the reduced outer shape of the wearer's head. In other embodiments, their shapes may differ, for example, the reduced inner wall of the cranial orthosis may be smaller in some regions, or may represent a less bulky shape of the head, wherein in these "reduced" regions the reduced inner wall of the cranial orthosis comprises flexible regions that may provide a firmer and more stable fit of the orthosis on the head, while at the same time, thanks to the flexibility, these regions support the growth of the head to a certain extent.

In the step of determining the reduced inner wall of the cranial orthosis, the shape of the reduced inner wall of the cranial orthosis may be designed by averaging the area of the model of the outer shape of the wearer's head with the area of the representation of the target outer shape of the wearer's head, and thus the 3D model of the reduced inner wall of the cranial orthosis is formed by points corresponding to the average of these coordinates of these two areas representing the initial and target shapes of the wearer's head. In other embodiments, the reduced inner wall may be designed such that it is not entirely halfway between these areas as in the case of the average but closer to the area forming the model of the initial outer shape of the wearer's head or to the area of the model of the target outer shape of the wearer's head, wherein it may preferably be located at 1/3 to 2/3 of the distance from the area of the initial model to the area of the target model of the wearer's head. More preferably, the shape of the reduced inner wall of the cranial orthosis is located 1/3 to 1/2 of the distance from the area of the initial model to the area of the target model of the wearer's head. In specific dimensions, the largest gap between the model of the outer shape of the wearer's head and the representation of the target shape of the wearer's head in the place of deformation often reaches a distance of 10 to 25 mm, wherein the reduced inner wall of the cranial orthosis may be at a distance of 3.33 mm to 5 mm in the corresponding region in the case of a 10 mm gap and 8.33 mm to 12.5 mm in the case of a 25 mm gap between the model of the outer shape of the wearer's head and the representation of the target shape of the wearer's head in the place of deformation. In the other regions, the distances between the model of the outer shape of the wearer's head and the representation of the target shape of the wearer's head decrease in the direction from the region of the greatest deformation and, according to the given ratio, the distances relative to the reduced inner wall of the cranial orthosis decrease accordingly.

The methods of designing the reduced inner wall of the cranial orthosis presented above in the preceding paragraph are adapted to support growth uniformly in all the regions of deformation where the symmetry of the head, and possibly also the proportions of the head, are corrected by growth into the target inner wall of the cranial orthosis. In other preferred embodiments, for combined defects in symmetry and proportion of the wearer's head, the reduced inner wall of the cranial orthosis may be designed such that it corresponds to the outer shape of the wearer's head with corrected symmetry, wherein the inner shell and the formed reduced inner cavity of the cranial orthosis first solve the symmetry of the head and then, after removal of the inner shell, the growth of the head into the target inner cavity of the cranial orthosis solves the proportion of the head. In other embodiments, first the proportions of the head and then the symmetry of the head may be solved analogously.

The step of designing the inner shell is based on the step of designing the outer shell and is based on the step of determining the reduced inner wall of the cranial orthosis and/or the step of determining the target inner wall of the cranial orthosis. As part of the design of the inner shell, the implementation of the respective outer shell for which the inner shell is designed is evaluated. In the step of designing the inner shell, it is evaluated whether any additional layers will be implemented on the inner shell, such as a lining. These layers and additional elements in the inner cavity of the orthosis affect the shape of the inner cavity of the cranial orthosis for insertion of the wearer's head, whether target or reduced, and therefore affect its function as a remodeling tool. All this information must be processed to create a customized lining and shell that together form the ideal inner wall of the cranial orthosis that will be both comfortable and will represent the ideal remodeling tool to achieve a healthy shape of the head. Thus, in some embodiments, the inner shell or surface thereof may correspond to the shape of the reduced inner wall of the cranial orthosis increased by a thickness corresponding to the given additional layer, such as a lining, such that together they form a shape corresponding to the reduced inner wall of the cranial orthosis. In other words, the surface of the inner shell does not have to correspond to the shape of the reduced inner wall of the cranial orthosis, wherein the resulting shape of the reduced inner wall of the cranial orthosis is achieved by placing an additional layer on the surface of the inner shell. In embodiments without additional layers, the shape of the surface of the inner shell may directly correspond to the reduced outer shape of the wearer's head.

In embodiments where the inner shell is connected to the inner wall of the outer shell that forms the target inner wall of the cranial orthosis, the structure of the inner shell is adapted to this wall such that, after connecting, the surface of the outer shell continuously follows the surface of the inner wall of the outer shell, while at the same time the surface of the outer shell corresponds to the corresponding reduced inner wall of the cranial orthosis.

In embodiments in which the cranial remodeling orthosis of the invention is a part of an assembly with at least two replaceable inner shells, these inner shells are designed such that both shells can be removably connected to one outer shell of the cranial orthosis. One such replaceable inner shell may be designed based on the reduced shape of the inner wall of the cranial orthosis, and the other such replaceable inner shell may be designed based on the target shape of the inner wall of the cranial orthosis. Thus, in these embodiments, the step of designing the inner shell comprises a design of at least two inner shells based on the step of determining the reduced inner wall of the cranial orthosis and the step of determining the target inner wall of the cranial orthosis.

Preferably, in the step of designing the inner shell, the inner shell is designed such that it is located exclusively in the space between the target inner wall of the cranial orthosis and the model of the outer shape of the wearer's head. The inner shells preferably serve mainly to reduce the space between the target inner wall of the cranial orthosis and the model of the outer shape of the wearer's head, wherein in other regions they would cause unnecessary pressure on the wearer. In this way, the inner shell can also be designed and produced additionally to already formed cranial orthoses that already form the target inner wall of the cranial orthosis.

Preferably, in the step of comparing, a digitized 3D model of the outer shape of the wearer's head and a digitized 3D model of the target inner wall of the cranial orthosis are compared. In this way, it is possible to easily compare these models and design the reduced inner wall of the cranial orthosis more accurately without major deviations.

Preferably, the method of designing and producing the cranial remodeling orthosis comprises the step of obtaining the input parameters, wherein the input parameter is the model of the outer shape of the wearer's head and at least one parameter from the following set: age, gender, type, and severity of the deformity, planned length of the cranial orthosis treatment, width of the head, length of the head, circumference of the head, circumference percentile of the growth of the head, and diagonal dimensions of the head, wherein in the step of designing the model of the cranial remodeling orthosis, the model of the cranial remodeling orthosis is designed based on the input parameters. Age determines, among other things, the size of the head, its physiological shape, and the rate of growth of the head. These data can be used to improve the accuracy of the models of the inner walls of the cranial orthosis, wherein the rate of growth of the head determines when the head will grow to both the reduced and target shape, allowing for precise timing of the remodeling process and follow-ups with the specialist. The planned length of the treatment, especially in combination with the rate of growth, forms the data on which the specific implementation of the inner walls of the cranial orthosis is based. The circumference percentile of the growth of the head is a datum determining the curve of growth of the head over time, which is again a datum that the specific implementation of the inner walls of the cranial orthosis can be adapted to. The type and severity of the deformity may also serve to improve the accuracy of the model of the cranial orthosis, which may thus be reflected in specific implementations of the inner walls of the cranial orthosis, specifically through the implementation of the lining, flexible regions, and/or distances between the inner walls of the cranial orthosis and the wearer's head in the regions of the deformation.

Preferably the step of producing the inner shell is carried out by a 3D printing method. The 3D printing method makes it possible to produce the inner shell of the invention at minimal initial cost.

Preferably, in the step of determining the reduced inner wall of the cranial orthosis, the reduced inner wall of the cranial orthosis is determined such that the volume between the model of the outer shape of the wearer's head and the reduced inner wall of the cranial orthosis is 10 to 90% smaller than the volume between the model of the outer shape of the wearer's head and the target inner wall of the cranial orthosis. More preferably, the volume between the model of the outer shape of the wearer's head and the reduced inner wall of the cranial orthosis is 40 to 60% smaller than the volume between the model of the outer shape of the wearer's head and the target inner wall of the cranial orthosis.

### Description of Drawings

A summary of the invention is further clarified using exemplary embodiments thereof, which are described with reference to the accompanying drawings, in which:
Fig. 1 shows a lateral section of the cranial remodeling orthosis according to the first exemplary embodiment of the invention,
Fig. 2 shows an upper section of the cranial remodeling orthosis according to the first exemplary embodiment of the invention,
Fig. 3 is a perspective illustration of the cranial remodeling orthosis according to the first exemplary embodiment of the invention with a handling opening and a perforated surface of the inner shell,
Fig. 4 is a perspective illustration of the cranial remodeling orthosis according to the first exemplary embodiment of the invention with an alternative implementation of the inner shell with the perforated surface of the inner shell without the handling opening,
Fig. 5 is a perspective illustration of the cranial remodeling orthosis according to the first exemplary embodiment of the invention with an alternative implementation of the inner shell without the perforated surface of the inner shell,
Fig. 6 shows a lateral section of the cranial remodeling orthosis according to the second exemplary embodiment of the invention,
Fig. 7 shows an upper section of the cranial remodeling orthosis according to the second exemplary embodiment of the invention,
Fig. 8 is a perspective illustration of the cranial remodeling orthosis according to the second exemplary embodiment of the invention,
Fig. 9 shows a lateral section of the cranial remodeling orthosis according to the third exemplary embodiment of the invention,
Fig. 10 shows an upper section of the cranial remodeling orthosis according to the third exemplary embodiment of the invention,
Fig. 11 is a perspective illustration of the cranial remodeling orthosis according to the third exemplary embodiment of the invention,
Fig. 12 shows a lateral section of the cranial remodeling orthosis according to the fourth exemplary embodiment of the invention,
Fig. 13 shows an upper section of the cranial remodeling orthosis according to the fourth exemplary embodiment of the invention,
Fig. 14 is a perspective illustration of the cranial remodeling orthosis according to the fourth exemplary embodiment of the invention,
Fig. 15 shows a lateral section of the cranial remodeling orthosis according to the fifth exemplary embodiment of the invention,
Fig. 16 shows an upper section of the cranial remodeling orthosis according to the fifth exemplary embodiment of the invention,
Fig. 17 is a perspective illustration of the cranial remodeling orthosis according to the fifth exemplary embodiment of the invention,
Fig. 18 shows a lateral section of the cranial remodeling orthosis according to the sixth exemplary embodiment of the invention,
Fig. 19 shows an upper section of the cranial remodeling orthosis according to the sixth exemplary embodiment of the invention,
Fig. 20 shows a lateral section of the cranial remodeling orthosis according to the seventh exemplary embodiment of the invention,
Fig. 21 shows an upper section of the cranial remodeling orthosis according to the seventh exemplary embodiment of the invention,
Fig. 22 is a perspective illustration of the cranial remodeling orthosis according to the seventh exemplary embodiment of the invention,
Fig. 23 shows a lateral section of the cranial remodeling orthosis according to the eighth exemplary embodiment of the invention, with the inner shell defining the reduced inner cavity of the cranial orthosis,
Fig. 24 shows an upper section of the cranial remodeling orthosis according to the eighth exemplary embodiment of the invention, with the inner shell defining the reduced inner cavity of the cranial orthosis,
Fig. 25 shows a lateral section of the cranial remodeling orthosis according to the eighth exemplary embodiment of the invention, with the inner shell defining the target inner cavity of the cranial orthosis,
Fig. 26 shows an upper section of the cranial remodeling orthosis according to the eighth exemplary embodiment of the invention, with the inner shell defining the target inner cavity of the cranial orthosis,
Fig. 27 shows a lateral section of the cranial remodeling orthosis, with the outer shell defining the target inner cavity of the cranial orthosis without the inner shell,
Fig. 28 shows an upper section of the cranial remodeling orthosis, with the outer shell defining the target inner cavity of the cranial orthosis without the inner shell,
Fig. 29 shows a diagram of the centered (initial) model of the outer shape of the wearer's head, the model of the reduced outer shape of the wearer's head, and the model of the target outer shape of the wearer's head.

### Exemplary Embodiments of the Invention

The cranial remodeling orthosis of the invention will be further clarified using exemplary embodiments with reference to the respective drawings.

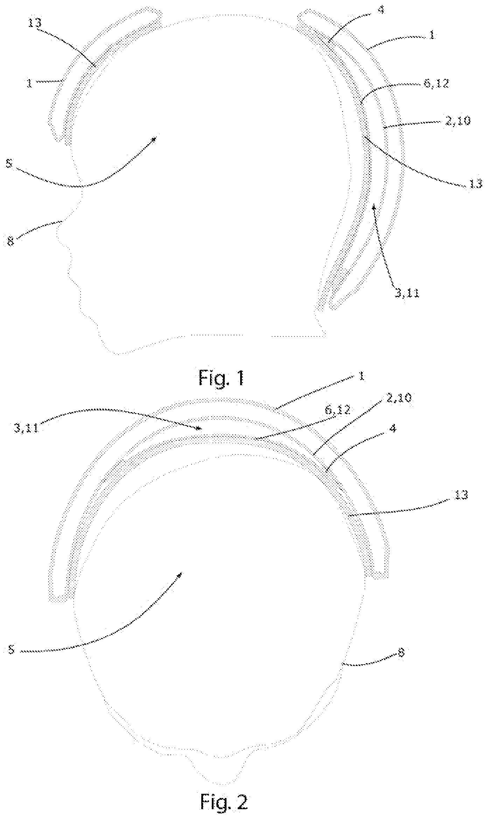

The first exemplary embodiment of the basic parts of the invention and its arrangement are illustrated in Fig. 1, Fig. 2, and Fig. 3. The cranial orthosis of the first exemplary embodiment comprises an outer shell 1 comprising an inner wall 2 of the outer shell defining an inner cavity of the cranial orthosis, where both the outer and inner shells 1,4 are made of a 3D printed material, in particular polyamide. The inner wall 2 of the outer shell here represents the target inner wall 10 of the cranial orthosis, wherein the target cavity of the cranial orthosis corresponds to the inner cavity 3 of the outer shell. The inner shell 4 is attached to the inner wall 2 of the outer shell by gluing. On the surface 6 of the inner shell and on the adjacent regions of the inner wall 2 of the outer shell, a lining 13 is placed, wherein the lining 13 and the inner shell 4 together reduce the volume of the inner cavity 3 of the outer shell to the reduced inner cavity 5 of the cranial orthosis. The inner shell 4 defines a space that reduces the target inner cavity 11 of the cranial orthosis (inner cavity 3 of the outer shell), wherein the resulting reduced inner wall 12 of the cranial orthosis is represented by the surface of the lining 13. In the design of the inner shell 4, the thickness of the lining 13 was considered such that when the lining 13 is inserted, together they form the resulting reduced inner wall 12 of the cranial orthosis. The inner shell 4 comprises the perforated surface 6 of the inner shell, including a handling opening 14 in the surface 6 of the inner shell that serves to facilitate the removal of the inner shell 4 from the outer shell 1. The cranial orthosis of the first exemplary is two-piece, wherein it comprises a front (left in Fig. 1) and a rear piece of the shell (right in Fig. 1), which together form the outer shell 1. The inner wall 2 of the outer shell on the front part is provided with the lining 13, wherein the inner wall 2 of the outer shell on the front piece and the corresponding lining 13 together partially form the target and reduced inner wall 10,12 of the cranial orthosis. Thus, in this embodiment of the invention, the difference between the target and reduced inner wall 10,12 of the cranial orthosis is formed only by the inner shell 4 and the lining 13 on the rear piece of the cranial orthosis.

The inner wall 2 of the outer shell of the rear piece may also be adapted with its shape for placing the lining 13, wherein together they may thus form the target inner wall 10 of the cranial orthosis on the rear piece of the outer shell 1. In such an embodiment, the lining 13 illustrated in Fig. 1 and 2 may, after the removal of the inner shell 4, simultaneously form the lining 13 that, together with the inner wall 2 of the outer shell of the rear piece, forms the target inner wall 10 of the cranial orthosis.

On a model 8 of the outer shape of the head illustrated in Fig. 2, it can be seen that the head comprises a significant deformation in the right rear part (top left in Fig. 2) that is reduced towards the left rear side of the head (top right in Fig. 2), wherein on the left rear side of the head it has nearly a physiological, target shape. As can be seen in Fig. 2, the reduced inner cavity 5 is designed based on the shape of the outer head of the wearer with corrected symmetry. The difference between the reduced inner wall 12 and the target inner wall 10 of the cranial orthosis is only in the length of the head, where the target inner wall 10 is slightly more recessed, their left and right sides are symmetrical, as can be seen in the upper section of Fig. 2. After achieving the shape of the wearer's head close to the shape of the reduced inner wall 12 of the cranial orthosis, a nearly corrected symmetry of the head is therefore expected. Therefore, the target inner wall 10 is then adapted to correct the proportions of the head.

In an alternative embodiment of the cranial orthosis of the first exemplary embodiment of the invention, the target inner wall 10 of the cranial orthosis, or the inner wall 2 of the outer shell, may be designed to be adapted to be provided with the lining 13, which is inserted into the cavity after the removal of the inner shell 4. In such an embodiment, the inner wall 2 of the outer shell is larger by a dimension corresponding to the given additional layer, such as the lining 13, such that when this layer is inserted together they form the resulting target inner wall 10 of the cranial orthosis.

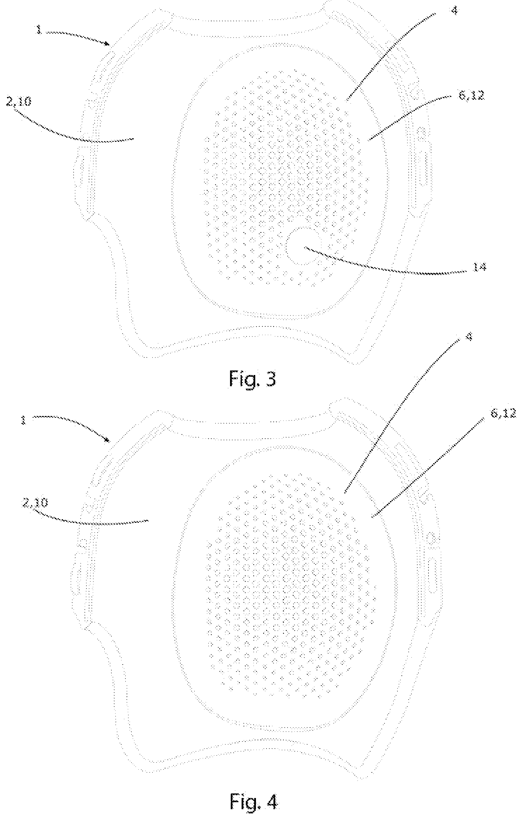

In an alternative embodiment of the cranial orthosis of the first exemplary embodiment of the invention, the inner shell 4 does not comprise the handling opening 14 in the surface 6 of the inner shell. Such an embodiment is illustrated in Fig. 4.

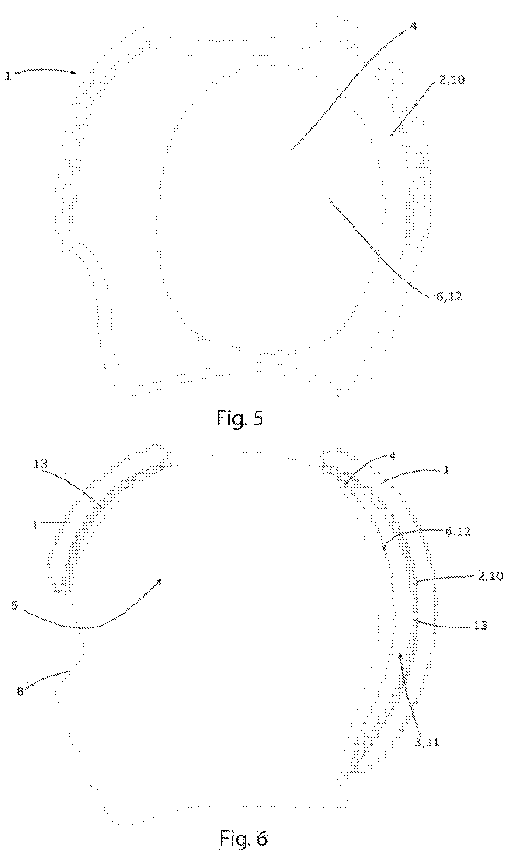

In an alternative embodiment of the cranial orthosis of the first exemplary embodiment of the invention illustrated in Fig. 5, the inner shell 4 does not comprise the perforated surface 6 of the inner shell.

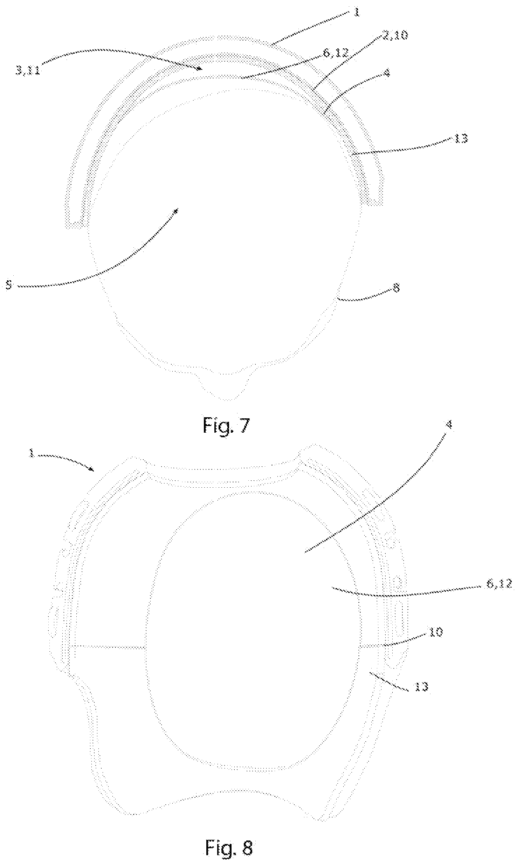

The cranial orthosis of the second exemplary embodiment is illustrated in Fig. 6 to Fig. 8. This embodiment differs from the first exemplary embodiment in that the inner shell 4 is connected to the outer shell 1 via the lining 13 that the outer shell 1 is provided with in the place of application of the inner shell 4. The inner shell 4 is connected to the lining 13 by double-sided adhesive tape. Thus, the target inner wall 10 of the cranial orthosis in this embodiment means the surface of the lining 13 on the inner side of the outer shell, and the reduced inner wall 12 of the cranial orthosis is formed by the surface 6 of the inner shell and the surface of the lining 13 on the inner wall 2 of the outer shell that is not covered by the inner shell 4.

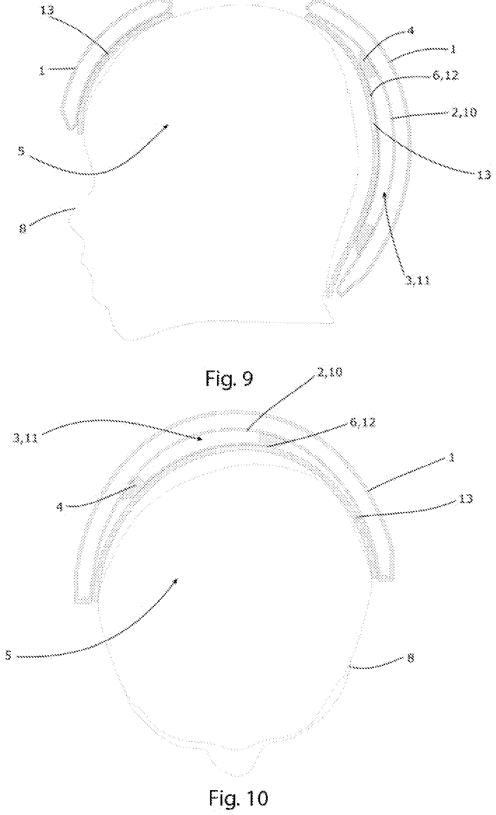

The cranial orthosis of the third exemplary embodiment is illustrated in Fig. 9 to Fig. 11. This embodiment differs from the first exemplary embodiment in that the reduced inner wall 12 of the cranial orthosis corresponds to a direct intermediate stage of growth between the initial outer shape of the wearer's head and the target outer shape 9 of the wearer's head. Thus, the area of the reduced inner wall 12 of the cranial orthosis corresponds to the average between the area of the model 8 of the outer shape of the wearer's head and the area of the model of the representation of the target outer shape 9 of the wearer's head. Thus, the reduced inner wall 12 of the cranial orthosis, or the reduced model 8 of the outer shape of the wearer, corresponds to a shape of the head showing both signs of asymmetry and defects in proportions, wherein both the symmetry and the proportions are corrected only by the target inner wall 10 of the cranial orthosis.

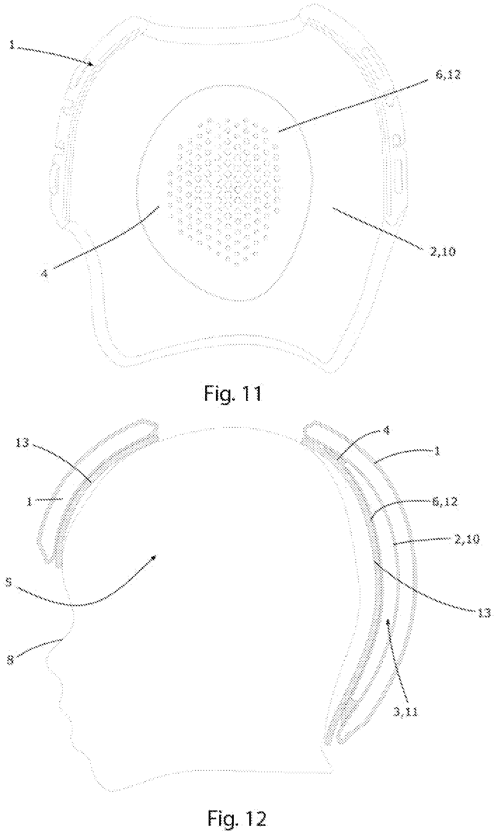

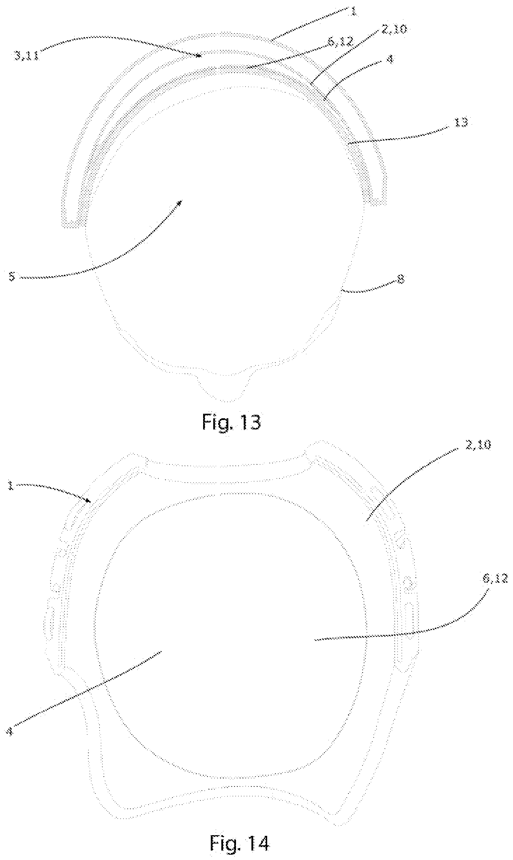

The cranial orthosis of the fourth exemplary embodiment is illustrated in Fig. 12 to Fig. 14. This embodiment differs from the third exemplary embodiment in that it comprises a larger inner shell 4, wherein the difference between the reduced inner cavity 5 and the target inner cavity 11 is larger than in the third exemplary embodiment. The surface 6 of the inner shell of the fourth exemplary embodiment follows more continuously the inner wall of the cranial orthosis, and the wearing of the cranial orthosis is thus more comfortable as the reduced inner wall 12 of the cranial orthosis better corresponds to the actual shape of the wearer's head. In this embodiment, the inner shell 4 is connected to the inner wall 2 of the outer shell with double-sided adhesive tape.

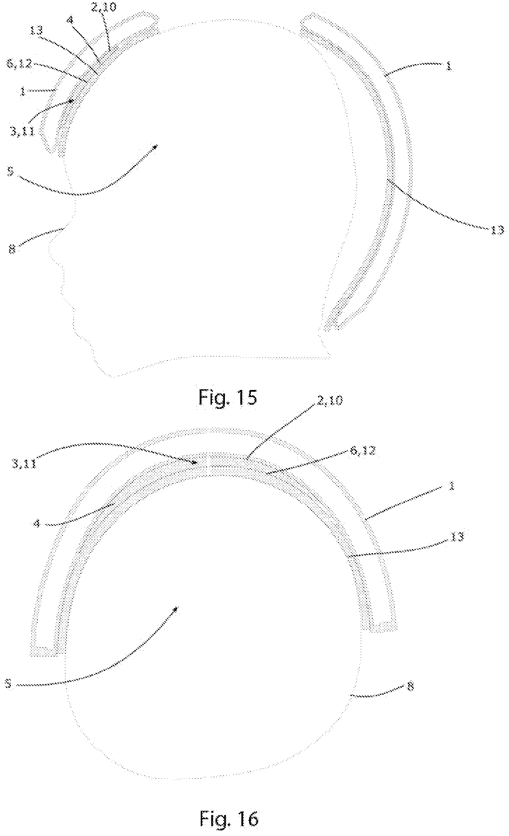

The cranial orthosis of the sixth exemplary embodiment is illustrated in Fig. 15 to Fig. 17. In this embodiment, the cranial remodeling orthosis is adapted to correct a deformation in the front part of the head. The inner shell 4 is connected to the inner wall 2 of the outer shell of the front piece. The inner shell 4 completely fills the space between the surface 6 of the inner shell and the inner wall 2 of the outer shell, wherein it is made of a 3D printed thermoplastic elastomer.

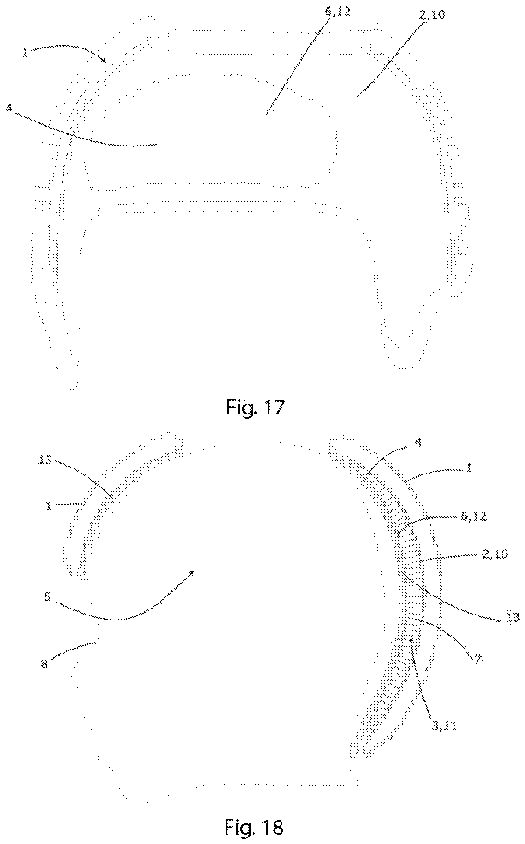

The cranial orthosis of the sixth exemplary embodiment is illustrated in Fig. 18 to Fig. 19. This embodiment differs from the first exemplary embodiment in that the inner shell 4 comprises a mesh 3D structure 7 filling the space between the surface 6 of the inner shell and the inner wall 2 of the outer shell. The inner shell 4 is made of a 3D printed thermoplastic elastomer, wherein it therefore has elastic properties, thereby further increasing the flexibility of the reduced inner wall 12 of the cranial orthosis that comes into contact with the wearer's head, making the cranial orthosis more comfortable.

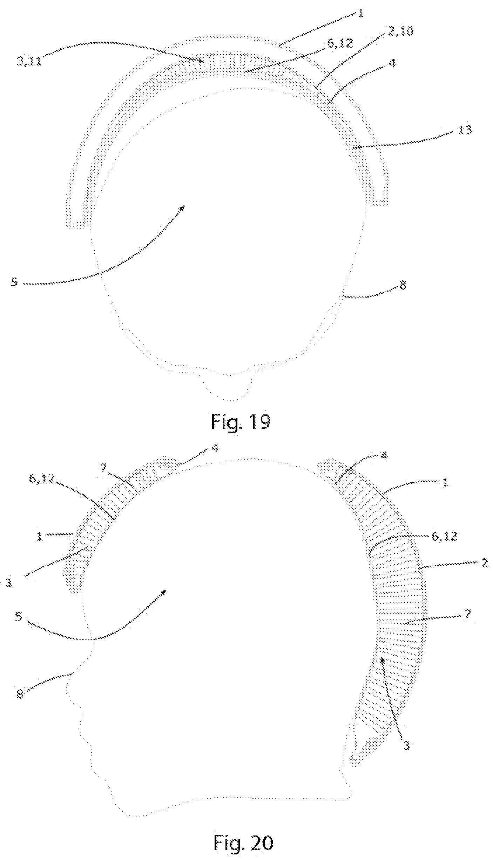

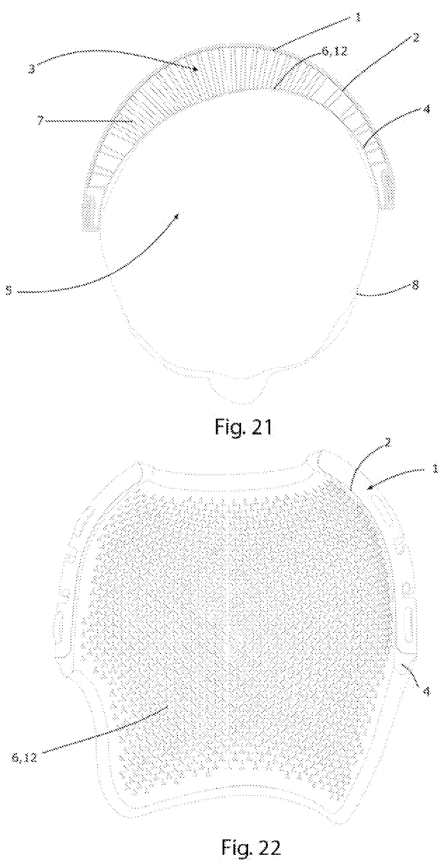

The cranial orthosis of the seventh exemplary embodiment is illustrated in Fig. 20 to Fig. 22. This cranial remodeling orthosis is particularly characterized in that it is a part of an arrangement of a cranial remodeling orthosis that comprises two different inner shells 4. Fig. 20 shows a section of the cranial remodeling orthosis of the seventh exemplary embodiment with the first inner shell 4 defining the reduced inner cavity 5 of the cranial orthosis. The first inner shell 4 is connected to the outer shell 1 along its margin by a mechanical connection of a groove-projection type, where the inner shell 4 is made by 3D printing from a thermoplastic elastomer, wherein it defines with its shape both the groove and the projection in the place of connection, the outer shell 1 in this place also defines with its shape both the groove and the projection corresponding to both the groove and the projection of the inner shell 4. The first inner shell 4 comprises the perforated surface 6 of the inner shell and the mesh 3D structure 7 following the surface 6 of the inner shell, which fills the space between the surface 6 of the inner shell and the inner wall of the cranial orthosis. In this embodiment, both the mesh 3D structure 7 and the surface 6 of the inner shell are formed by an elastic thermoplastic elastomer, wherein the entire inner shell 4 represents a flexible region that elastically deforms when a force acts on the surface 6 of the inner shell. In alternative embodiments, the mesh 3D structure 7 is formed of a more rigid material, wherein it forms a support for the surface 6 of the inner shell. The model 8 of the outer shape of the wearer's head illustrated in Fig. 20 and 21 is the reduced outer shape of the wearer's head, where this model 8 corresponds to the reduced inner wall 12 of the cranial orthosis, thus illustrating the model 8 of the head at a moment when the head has grown into the reduced inner cavity 5 of the cranial orthosis and it is approximately time to replace the first inner shell 4 with the second inner shell 4 that defines the target inner cavity 11 of the cranial orthosis.

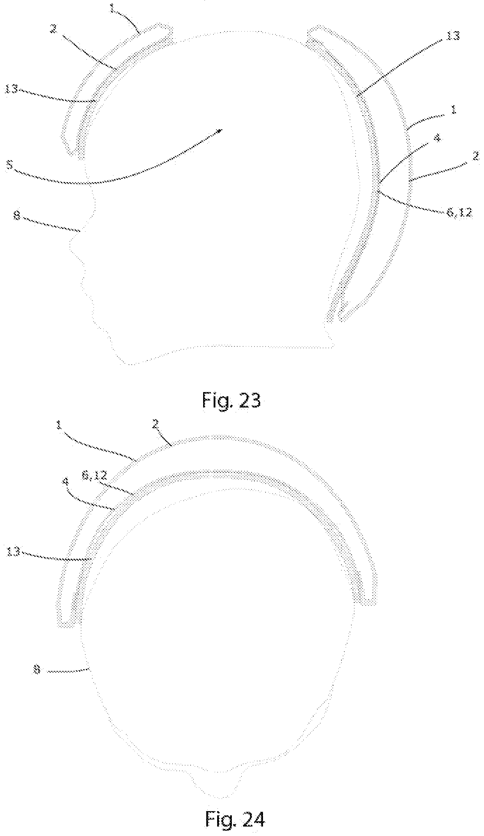

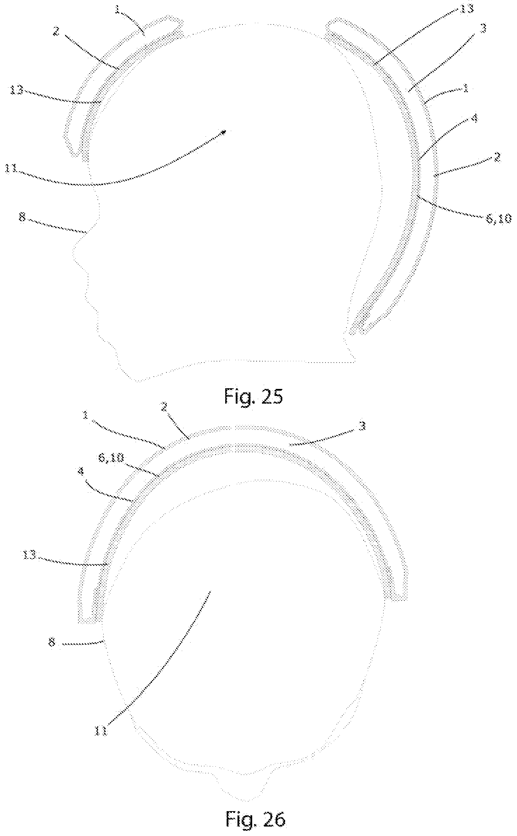

The cranial orthosis of the eighth exemplary embodiment is illustrated in Fig. 23 to Fig. 26. Like the seventh exemplary embodiment of the invention, the eighth embodiment is characterized in that it is a part of an assembly of a cranial remodeling orthosis comprising the first inner shell 4 defining the reduced inner wall 12 of the cranial orthosis and the second inner shell 4 defining the target inner wall 10 of the cranial orthosis. Both inner shells 4 are adapted for placement of the lining 13 on their surface, wherein they form the inner wall of the cranial orthosis together with this lining 13. The cranial orthosis of the eighth exemplary embodiment with the first inner shell 4 is illustrated in Fig. 23 and 24. The first inner shell 4 is formed by a surface wall of the inner shell 4 that defines the surface 6 of the inner shell and that is adjacent along its circumference to a corresponding area formed by the structure of the outer shell 1, wherein these areas of the inner and outer shells 4,1 are connected via double-sided adhesive tape. In an alternative embodiment, they are connected mechanically by a pin-and-groove connection. The surface of the first inner shell 4 is adapted with its shape to form the reduced inner wall 12 of the cranial orthosis together with the lining 13. The cranial orthosis of the eighth exemplary embodiment with the second inner shell 4 is illustrated in Fig. 25 and 26. The second inner shell 4 is structurally identical to the first inner shell 4, except that it differs in the shape of the surface of the second inner shell 4. It is because the surface of the second inner shell 4 is adapted with its shape to form the target inner wall 10 of the cranial orthosis together with the lining 13.

All the drawings of the eighth embodiment of the invention (Fig. 23 to Fig. 26) also show the initial model 8 of the outer shape of the wearer's head, wherein it can be observed how the first and second inner shells 4 change the size of the space between the initial outer shape of the wearer's head and the given inner wall of the cranial orthosis.

The method of designing and producing the cranial remodeling orthosis of the invention will be described herein using particular embodiments.

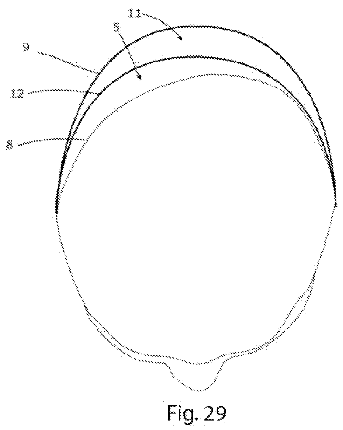

In an exemplary embodiment of the method of designing and producing the cranial remodeling orthosis of the invention, in the step of obtaining the model 8 of the outer shape of the wearer's head, the wearer's head is scanned by a scanning device, wherein the created 3D scan is stored in a memory of a computer device. This 3D scan of the wearer's head represents the initial model 8 of the outer shape of the wearer's head. The description of the relationship between the individual digitized areas (models 8 of the outer shape of the wearer's head, areas corresponding to the inner walls of the cranial orthosis, ...) is related to their centered position relative to the initial model 8 of the outer shape of the wearer's head, a schematic example of the centering of these areas is illustrated in Fig. 29.

The input parameters further comprise information that the target inner wall 10 of the cranial orthosis is to be provided with a 5 mm lining 13 and information that correction of the symmetry of the head is preferred, followed by correction of the proportions of the head. Based on this model 8 of the outer shape of the wearer's head, the target outer shape 9 of the wearer's head is further designed, which is then modified to the final implementation of the target inner wall 10 of the cranial orthosis. Now, the shape of the target inner wall 10 of the cranial orthosis is known, and the design of the outer shell 1 on the computer device follows such that the cranial orthosis in the result has the target shape of the inner wall of the cranial orthosis. The lining 13 with a thickness of 3 millimeters will be placed on the inner wall of the cranial orthosis, the inner wall 2 of the outer shell, which together with the lining 13 is to define the target inner cavity 11 of the cranial orthosis, is therefore increased by 3 mm over the area defining the target inner cavity 11 of the cranial orthosis, wherein the difference between the area defining the target inner cavity 11 of the cranial orthosis and the area defining the inner wall 2 of the outer shell corresponds to the dimensions of the lining 13. Thus, the lining 13 placed on the inner wall of the cranial orthosis reduces the inner cavity 3 of the outer shell to a cavity corresponding to the target inner cavity 11 of the cranial orthosis. Thus, it is now possible to design the entire inner shell 4 that is structurally designed from the determined shape of the inner wall 2 of the outer shell so as to give to the cranial orthosis sufficient firmness and stability of the structure of the cranial orthosis. Subsequently, the outer shell 1 is produced by the 3D printing method.

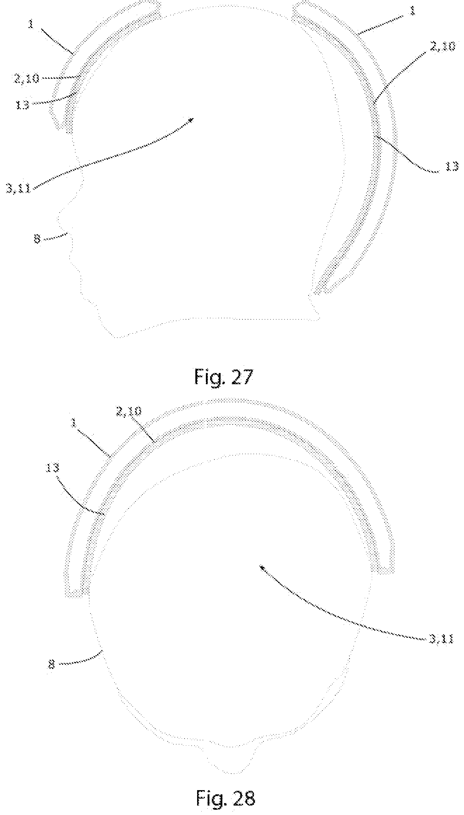

The produced outer shell 1 defining the target inner cavity 11 of the cranial orthosis is illustrated in Fig. 27 and 28, where a significant gap can be seen between the initial model 8 of the outer shape of the wearer's head and the target inner wall 10 of the cranial orthosis, resulting in unwanted rotations of the cranial orthosis around the wearer's head. Therefore, subsequently the inner shell 4 is designed that reduces this volume, and it is based on the reduced outer shape of the wearer's head, or the reduced inner wall 12 of the cranial orthosis.

The step of determining the reduced inner wall 12 of the cranial orthosis follows, which is schematically illustrated in Fig. 29. The step of determining the reduced inner wall 12 of the cranial orthosis comprises the step of comparing the model 8 of the outer shape of the wearer's head with the representation of the target outer shape 9 of the wearer's head, where the initial outer shape of the wearer's head is first compared with the target outer shape 9 of the wearer's head. In Fig. 29, it can be seen that the wearer's head has a significant deformation in the right rear region of the head, this deformation disturbs the overall symmetry of the head and its proportions. The reduced inner wall 12 of the cranial orthosis is to correspond to the shape of the head with corrected symmetry, wherein subsequently the defect in proportion will be addressed by growing the head into the target inner cavity 11 of the cranial orthosis. In this embodiment, the reduced inner wall 12 of the cranial orthosis is formed by modifying the target inner wall 10 of the cranial orthosis, which is modified by displacing it toward the model of the initial outer shape of the wearer's head while maintaining a symmetrical shape. The reduced inner wall 12 of the cranial orthosis is designed here such that it divides the volume between the area corresponding to the initial outer shape of the wearer's head and the area corresponding to the target outer shape 9 of the wearer's head into two approximately equal cavities (see Fig. 29).

The step of designing the inner shell 4 based on the reduced inner wall 12 of the cranial orthosis follows. The inner shell 4 will not be provided with the lining 13 or any other complementary layer, wherein the surface 6 of the inner shell is to correspond as closely as possible to the surface of the reduced inner wall 12 of the cranial orthosis. Thus, the inner shell 4 is designed such that its surface completely corresponds to the reduced inner wall 12 of the cranial orthosis in the place where it differs from the target inner wall 10 of the cranial orthosis; in the places where they overlap, the reduced inner wall 12 of the cranial orthosis is formed by the lining 13 on the inner wall 2 of the outer shell. The inner shell 4 is adapted for connection to the outer shell 1 via its lining 13, wherein it comprises a contact area around its circumference that corresponds to the lining 13 on the outer shell 1 in the given place, wherein they are connected by double-sided adhesive tape.

Subsequently, the designed inner shell 4 is produced by 3D printing and connected to the outer shell 1 in front of the lining 13 with double-sided adhesive tape, thus completing the step of producing the cranial orthosis.

The resulting product of the exemplary method of designing and producing according to the invention is a cranial orthosis according to the second exemplary embodiment illustrated in Fig. 6 to 8.

In the example of the method of designing and producing above, it can be seen that the step of designing and producing the outer shell 1 and the step of designing and producing the inner shell 4 can occur separately at different times. In an alternative embodiment, the inner shell 4 may be designed first and subsequently a suitable outer shell 1 may be designed therefor. This may, for example, be preferred in the methods of designing and producing the cranial orthosis of the seventh and eighth embodiments, which are a part of the assembly of the cranial orthosis with two inner shells 4. In these, based on the determined reduced and target inner walls 12,10 of the cranial orthosis, it is required to design the first and second inner shells 4 that are adapted for the same removable connection to the outer shell 1, then the outer shell 1 may be designed that represents the rest of the structure of the cranial orthosis providing the structural stability and firmness of the cranial orthosis.

### Industrial Applicability

The invention can be used in the field of remodeling orthoses.

### List of Reference Signs

- 1 -: Outer shell
- 2 -: Inner wall of the outer shell
- 3 -: Inner cavity of the outer shell
- 4 -: Inner shell
- 5 -: Reduced inner cavity of the cranial orthosis
- 6 -: Surface of the inner shell
- 7 -: Mesh 3D structure
- 8 -: Model of the outer shape of the head
- 9 -: Target outer shape of the head
- 10 -: Target inner wall of the cranial orthosis
- 11 -: Target inner cavity of the cranial orthosis
- 12 -: Reduced inner wall of the cranial orthosis
- 13 -: Lining
- 14 -: Handling opening

## Claims

1. A cranial remodeling orthosis comprising an outer shell (1) and an inner shell (4), wherein the inner wall (2) of the outer shell defines an inner cavity (3) of the outer shell for insertion of the wearer's head, wherein the inner shell (4) is removably connected to the outer shell (1) and defines a reduced inner cavity (5) for insertion of the wearer's head, which has a smaller volume than the inner cavity (3) of the outer shell, wherein the reduced inner cavity (5) corresponds to a certain intermediate stage of growth between an initial outer shape of the wearer's head and a target outer shape (9) of the wearer's head, **characterized in that** the inner shell (4) is removably connected to the outer shell (1) in at least 2 different places distributed around a margin of the inner shell (4).

2. The cranial remodeling orthosis according to claim 1, **characterized in that** the inner shell (4) is removably connected to the inner wall (2) of the outer shell.

3. The cranial remodeling orthosis according to claims 1 or 2, **characterized in that** it is a part of an assembly of the cranial remodeling orthosis that further comprises at least one other inner shell (4) for removable connection to the outer shell (1), wherein each of the at least two inner shells (4) defines a different reduced inner cavity (5) having a different volume.

4. The cranial remodeling orthosis according to claims 1 to 3, **characterized in that** the inner shell (4) comprises a perforated surface (6) of the inner shell.

5. The cranial remodeling orthosis according to claims 1 to 4, **characterized in that** the inner shell (4) is made of a 3D printed material.

6. The cranial remodeling orthosis according to claims 1 to 5, **characterized in that** the inner shell (4) further comprises a mesh 3D structure (7) between the surface (6) of the inner shell and the inner wall (2) of the outer shell.

7. The cranial remodeling orthosis according to claims 1 to 6, **characterized in that** the inner shell (4) is removably connected to the outer shell (1) along most of its margin.

## Patentansprüche

1. Eine kraniale remodellierende Orthese, umfassend eine Außenschale (1) und eine Innenschale (4), wobei die Innenwand (2) der Außenschale einen Innenhohlraum (3) der Außenschale zum Einführen des Kopfes des Trägers definiert, wobei die Innenschale (4) lösbar mit der Außenschale (1) verbunden ist und einen reduzierten Innenhohlraum (5) zum Einführen des Kopfes des Trägers definiert, der ein kleineres Volumen als der Innenhohlraum (3) der Außenschale aufweist, wobei der reduzierte Innenhohlraum (5) einer bestimmten Zwischenstufe des Wachstums zwischen einer Anfangsaußenform des Kopfes des Trägers und einer Zielaußenform (9) des Kopfes des Trägers entspricht, **dadurch gekennzeichnet, dass** die Innenschale (4) an mindestens 2 verschiedenen Stellen, die um einen Rand der Innenschale (4) verteilt sind, lösbar mit der Außenschale (1) verbunden ist.

2. Die kraniale remodellierende Orthese nach Anspruch 1, **dadurch gekennzeichnet, dass** die Innenschale (4) lösbar mit der Innenwand (2) der Außenschale verbunden ist.

3. Die kraniale remodellierende Orthese nach Anspruch 1 oder 2, **dadurch gekennzeichnet, dass** sie Teil einer Anordnung der kranialen remodellierenden Orthese ist, die ferner mindestens eine weitere Innenschale (4) zur lösbaren Verbindung mit der Außenschale (1) umfasst, wobei jede der mindestens zwei Innenschalen (4) einen ein unterschiedliches Volumen aufweisenden unterschiedlichen reduzierten Innenhohlraum (5) definiert.

4. Die kraniale remodellierende Orthese nach Ansprüchen 1 bis 3, **dadurch gekennzeichnet, dass** die Innenschale (4) eine perforierte Oberfläche (6) der Innenschale umfasst.

5. Die kraniale remodellierende Orthese nach Ansprüchen 1 bis 4, **dadurch gekennzeichnet, dass** die Innenschale (4) aus einem 3D-gedruckten Material hergestellt ist.

6. Die kraniale remodellierende Orthese nach Ansprüchen 1 bis 5, **dadurch gekennzeichnet, dass** die Innenschale (4) ferner eine 3D-Netzstruktur (7) zwischen der Oberfläche (6) der Innenschale und der Innenwand (2) der Außenschale umfasst.

7. Die kraniale remodellierende Orthese nach Ansprüchen 1 bis 6, **dadurch gekennzeichnet, dass** die Innenschale (4) entlang des größten Teils ihres Randes lösbar mit der Außenschale (1) verbunden ist.

## Revendications

1. Une orthèse de remodelage crânien comprenant une coque extérieure (1) et une coque intérieure (4), où une paroi intérieure (2) de la coque extérieure définit une cavité intérieure (3) de la coque extérieure pour l'insertion de la tête du porteur, où la coque intérieure (4) est reliée de manière amovible à la coque extérieure (1) et définit une cavité intérieure réduite (5) pour l'insertion de la tête du porteur, qui a un volume plus petit que la cavité intérieure (3) de la coque extérieure, où la cavité intérieure réduite (5) correspond à un certain stade intermédiaire de croissance entre une forme extérieure initiale de la tête du porteur et une forme extérieure cible (9) de la tête du porteur, **caractérisée en ce que** la coque intérieure (4) est reliée de manière amovible à la coque extérieure (1) en au moins 2 endroits différents répartis autour d'un bord de la coque intérieure (4).

2. L'orthèse de remodelage crânien selon la revendication 1, **caractérisée en ce que** la coque intérieure (4) est reliée de manière amovible à la paroi intérieure (2) de la coque extérieure.

3. L'orthèse de remodelage crânien selon les revendications 1 ou 2, **caractérisée en ce qu'**elle fait partie d'un ensemble d'orthèses de remodelage crânien qui comprend en outre au moins une autre coque intérieure (4) destinée à être reliée de manière amovible à la coque extérieure (1), où chacune des au moins deux coques intérieures (4) définit une cavité intérieure réduite différente (5) ayant un volume différent.

4. L'orthèse de remodelage crânien selon les revendications 1 à 3, **caractérisée en ce que** la coque intérieure (4) comprend une surface perforée (6) de la coque intérieure.

5. L'orthèse de remodelage crânien selon les revendications 1 à 4, **caractérisée en ce que** la coque intérieure (4) est fabriquée à partir d'un matériau imprimé en 3D.

6. L'orthèse de remodelage crânien selon les revendications 1 à 5, **caractérisée en ce que** la coque intérieure (4) comprend en outre une structure 3D maillée (7) entre la surface (6) de la coque intérieure et la paroi intérieure (2) de la coque extérieure.

7. L'orthèse de remodelage crânien selon les revendications 1 à 6, **caractérisée en ce que** la coque intérieure (4) est reliée de manière amovible à la coque extérieure (1) sur la majeure partie de son bord.
